(19) Europäisches Patentamt
European Patent Office
Office européen des brevets

(11) Veröffentlichungsnummer: **0 069 293**
**A1**

(12) # EUROPÄISCHE PATENTANMELDUNG

(21) Anmeldenummer: 82105600.9

(22) Anmeldetag: 25.06.82

(51) Int. Cl.³: **C 07 C 103/38**
C 07 C 103/48, C 07 D 307/32
C 07 D 317/28, C 07 D 319/00
A 01 N 37/22, A 01 N 37/46
A 01 N 43/08, A 01 N 43/24

(30) Priorität: 02.07.81 DE 3126082

(43) Veröffentlichungstag der Anmeldung: 12.01.83 Patentblatt 83/2

(84) Benannte Vertragsstaaten: AT BE CH DE FR GB IT LI NL SE

(71) Anmelder: BASF Aktiengesellschaft
Carl-Bosch-Strasse 38
D-6700 Ludwigshafen(DE)

(72) Erfinder: Eicken, Karl, Dr.
Waldstrasse 63
D-6706 Wachenheim(DE)

(72) Erfinder: Rentzea, Costin, Dr.
Neuenheimer Landstrasse 72
D-6900 Heidelberg(DE)

(72) Erfinder: Pommer, Ernst-Heinrich, Dr.
Berliner Platz 7
D-6703 Limburgerhof(DE)

(72) Erfinder: Ammermann, Eberhard, Dr.
Sachsenstrasse 3
D-6700 Ludwigshafen(DE)

(54) **Glykoletheressigsäurenaphthylamide, Verfahren zu ihrer Herstellung und diese enthaltende Fungizide.**

(57) Glykoletheressigsäurenaphthylamid der Formel

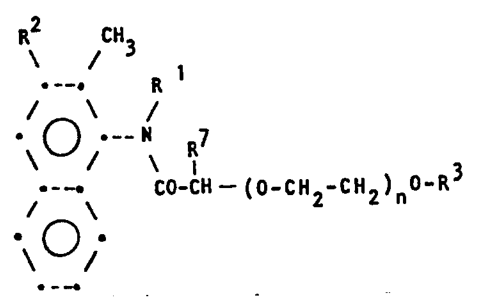

1

worin

$R^1$ $CH(CH_3)CO_2R^4$ oder

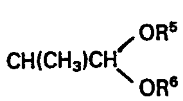

oder

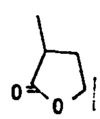

$R^2$ Wasserstoff oder Methyl

$R^3$ Alkyl

$R^4$ $C_1$-bis $C_3$-Alkyl

$R^5$ und $R^6$ unabhängig voneinander Alkyl oder zusammen eine gegebenenfalls substituierte Alkylenkette

$R^7$ Wasserstoff oder Methyl.

n 1 oder 2

bedeuten und diese Verbindungen enthaltende Fungizide.

EP 0 069 293 A1

# Glykoletheressigsäurenaphthylamide, Verfahren zu ihrer Herstellung und diese enthaltende Fungizide

Die vorliegende Erfindung betrifft neue Glykoletheressigsäurenaphthylamide, Verfahren zu ihrer Herstellung und diese enthaltende Fungizide.

Es ist bekannt, daß Trichlormethylthiophthalimid ein gutes Fungizid zur Bekämpfung von phytopatogenen Pilzen, insbesondere zur Bekämpfung von falschem Mehltau der Reben ist (Chemical Week 1972, Juni 21, S. 63). Es ist ferner bekannt, den N-(2,6-Dimethylphenyl-N-furyl-(2)-alaninsäuremethylester als Fungizid zu verwenden (DE-OS 25 13 732, DE-OS 25 13 788).

Es wurde nun gefunden, daß neue substituierte Glykoletheressigsäurenaphthylamide der Formel

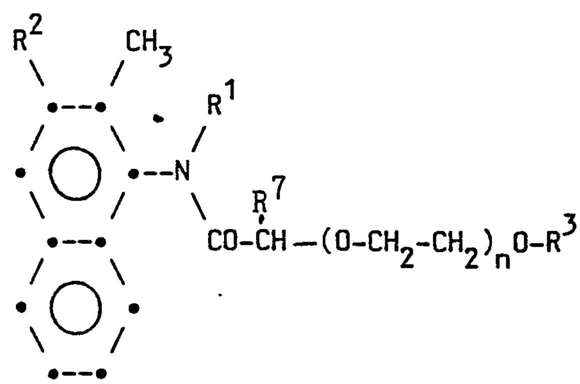

I

worin

$R^1$ $CH(CH_3)CO_2R^4$ oder 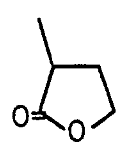

$R^2$ Wasserstoff oder Methyl

Sws/P

$R^4$ $C_1$- bis $C_3$-Alkyl
$R^7$ Wasserstoff oder Methyl
n 1 oder 2

bedeutet, wobei wenn n 1 ist, $R^3$ $C_3$- bis $C_6$-Alkyl ist oder wenn n 2 ist, $R^3$ $C_1$- bis $C_6$-Alkyl ist
$R^1$ bedeutet ferner $CH(CH_3)CH{<}^{OR^5}_{OR^6}$, in diesem Fall bedeutet $R^2$ Wasserstoff oder Methyl,
$R^3$ $C_1$- bis $C_6$-Alkyl
$R^5$ und $R^6$ unabhängig voneinander, $C_1$- bis $C_6$-Alkyl oder zusammen eine gegebenenfalls mit bis zu 2 Methyl- oder Ethylresten substituierte Alkylenkette mit 2 oder 3 C-Atomen im Alkylenrest, $R^7$ Wasserstoff oder Methyl und n bedeutet 1 oder 2, eine starke fungizide Wirkung haben.

Die Verbindungen enthalten im $-CH(CH_3)$-Rest ein chirales C-Atom und sind daher optisch aktiv. Die vorliegende Erfindung umfaßt sowohl die reinen optisch aktiven Verbindungen als auch ihre Mischungen.

Unter Alkyl oder Alkylrest einer Alkoxigruppe bei den Resten $R^3$, $R^4$, $R^5$ oder $R^6$ sind je nach Zahl der genannten Kohlenstoffatome beispielsweise folgende Gruppen zu verstehen: Methyl, Ethyl, n-Propyl, iso-Propyl, n-, iso-, sec.- oder tert.-Butyl, Pentyl oder Hexyl und ihre Isomeren.

Unter der gegebenenfalls substituierten Alkylenkette ist z.B. zu verstehen: Ethylen, 1,3-Propylen, 1,2-Propylen, 1,2-Butylen, 1,3-Butylen, 2,3-Butylen.

Die Verbindungen der Formel I können durch Umsetzung einer Verbindung der Formel

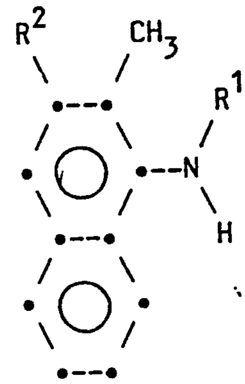

II

mit einer Säure der Formel

$$HO-CO-\overset{R^7}{\overset{|}{C}}H-(O-CH_2-CH_2)_n O-R^3 \qquad III$$

oder ihrem Säurehalogenid, wobei $R^1$ bis $R^7$ und n die oben angegebenen Bedeutungen haben, hergestellt werden. Als Lösungsmittel kommen z.B. aliphatische oder aromatische Kohlenwasserstoffe wie Toluol, Xylol, Petrolether; halogenierte Kohlenwasserstoffe wie Chlorbenzol, Methylenchlorid, Chloroform, Ethylenchlorid; Ether wie Diethylether, Tetrahydrofuran, Dioxan; Nitrile wie Acetonitril; N,N-dialkylierte Amide wie Dimethylformamid; Ketone wie Aceton; aliphatische Ester wie Essigester oder Gemische dieser Lösungsmittel in Frage.

Die Reaktionstemperaturen liegen beispielsweise zwischen -10°C und +180°C, vorzugsweise zwischen 20 und 120°C. In einigen Fällen ist die Verwendung von säurebindenden Mitteln vorteilhaft. Als solche kommen tertiäre Amine, wie Trialkylamine (z.B. Triethylamin), Pyridin und Pyridinbasen oder anorganische Basen wie Oxide, Hydroxide, Hydrogencarbonate und Carbonate von Alkali- und Erdalkalimetallen in Betracht.

Die Herstellung der Vorprodukte der Formel II kann nach Methoden vorgenommen werden, wie sie für die Herstellung von Anilino-alkansäureestern bekannt sind (J. Org. Chem. 30, 4101 (1965); Tetrahedron 1967, 487, 493). Zur Herstellung der neuen Glykolether-acetanilide der Formel I setzt man auf 1 Mol Anilino-alkansäureester der Formel II zweckmäßig mindestens 1 Mol, vorzugsweise 1,1 bis 1,2 Mol Säure der Formel III bzw. ihrer Säurederivate ein. Die Glykolether-essigsäuren sind teilweise bekannte Verbindungen (Chem. Ber. 63, 3117 (1930), US 2 769 838) oder können nach bekannten Methoden hergestellt werden.

Zur Herstellung der Verbindungen der Formel I wird beispielsweise nach Entfernen des entstandenen Nebenproduktes, z.B. eines Halogenids, ggf. nach Waschen mit Wasser, das Lösungsmittel durch Verdampfen entfernt. Das erhaltene Produkt der Formel I ist in vielen Fällen rein, wenn nötig kann es z.B. durch Destillation gereinigt werden.

Verbindungen der Formel I, in denen $R^1$ für $CH(CH_3)CH<^{OR^5}_{OR^6}$ steht und $R^5$ und $R^6$ zusammen eine gegebenenfalls durch bis zu 2 Methyl oder Ethyl substituierte Alkylenkette bedeuten, können z.B. durch Umsetzung von Verbindungen der Formel

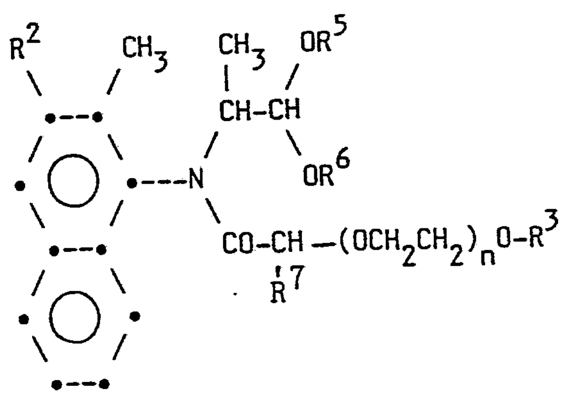

IV

in der $R^2$, $R^3$, $R^5$, $R^6$, $R^7$ und n die oben angegebene Bedeutung haben, mit 1,2- oder 1,3-Diolen bei Temperaturen von $0^o$ bis $180^oC$, vorzugsweise $20^o$ bis $120^oC$ hergestellt werden. Ein Verdünnungsmittel kann gegebenenfalls zugesetzt werden. Besonders vorteilhaft ist es, einen Reaktionsbeschleuniger wie Lewis-Säuren oder Protonensäuren, beispielsweise $ZnCl_2$, $AlCl_3$, $BF_3$ oder Mineralsäure oder Sulfonsäuren zuzusetzen. Der freigesetzte niedere Alkohol wird durch Destillation gegebenenfalls i. Vakuum entfernt.

Die folgenden Beispiele erläutern die Herstellung der neuen Glykoletheressigsäurenaphthylamide der Formel I. In den Beispielen verhalten sich Gewichtsteile zu Volumenteilen wie Kilogramm zu Liter.

Beispiel 1

12,5 Gewichtsteile N-(2-Methylnapth-1-yl)-alaninsäuremethylester und 10,8 Gewichtsteile 3,6,9-Trioxa-undecansäurechlorid wurden in 70 Volumenteilen trockenem Toluol unter Durchleiten von trockenem Stickstoff 4 Std. am Rückfluß erhitzt. Nach dem Abkühlen wurde die Lösung mit 10 %iger Soda-Lösung und dreimal mit Wasser gewaschen und getrocknet. Aus dem Filtrat isolierte man nach dem Verdampfen des Toluols ($70^oC$/0,1 mbar) 14,7 Gewichtsteile N-(1-Methoxicarbonylethyl)-N-3,6,9-trioxaundecansäure-2--methyl-napth-1-yl-amid ($n_D^{25}$= 1,5445) (Verbindung 8).

$C_{23}H_{31}NO_6$ M 417

| | C | H | N |
|---|---|---|---|
| ber. | 66,17 | 7,48 | 3,34 |
| gef. | 66,6 | 7,5 | 2,9 |

Beispiel 2

24,6 Gewichtsteile N-(1,1-Dimethoxi-prop-2-yl-)-2-methyl--napth-1-yl-amin, 16,0 Gewichtsteile 3,6-Dioxa-heptansäurechlorid, 12,6 Gewichtsteile Natriumhydrogencarbonat und 12,5 Gewichtsteile wasserfreies Natriumsulfat würden in 150 Volumenteilen Toluol 1 Std. bei 25°C und 3 Std. bei 80°C gerührt. Nach dem Abkühlen wurde die Reaktionsmischung dreimal mit je 50 Volumenteilen Wasser gewaschen und über $Na_2SO_4$ getr. Durch Verdampfen des Lösungsmittels erhielt man 32,5 Gewichtsteile N-(1,1-Dimethoxi-prop-2-yl)-N-3,6--dioxaheptansäure-2-methyl-napth-1-yl-amid. ($n_D^{25}$: 1,5554) (Verbindung 18).

$C_{21}H_{29}NO_5$ M 375

| | C | H | N |
|---|---|---|---|
| ber. | 67,18 | 7,79 | 3,73 |
| gef. | 67,6 | 7,8 | 3,8 |

Beispiel 3

20,0 Gewichtsteile N-(1,1-Dimethoxi-prop-2-yl)-N-3,6-dioxaheptansäure-2-methylnapth-1-yl-amid 3,8 g Glykol, 0,1 g p-Toluolsulfonsäure wurden unter Überleiten von Stickstoff 1 Std. auf 110°C (Innentemp.) erhitzt bis kein Methanol mehr abdestillisiert. Das Reaktionsgemisch wurde zwischen Ether und Wasser verteilt und die Etherphase dreimal mit Wasser gewaschen und getrocknet. Durch Verdampfen des Ethers im Vakuum erhielt man 10,8 Gewichtsteile der entsprechenden Ethylenketalverbindung ($n_D^{25}$: 1,5679) (Verbindung 24):

$C_{21}H_{27}NO_5$

| | | | | |
|---|---|---|---|---|
| 16 | 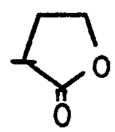 | H | $n-C_3H_7$ | 2 |
| 17 | 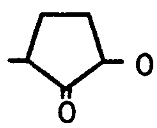 | H | $n-C_4H_9$ | 2 |
| 19 | $CH(CH_3)CH(OCH_3)_2$ | H | $C_2H_5$ | 1 |
| 20 | $CH(CH_3)CH(OCH_3)_2$ | H | $nC_3H_7$ | 1 |
| 21 | $CH(CH_3)CH(OCH_3)_2$ | H | $n-C_4H_9$ | 1 |
| 22 | $CH(CH_3)CH(OCH_3)_2$ | H | $CH_3$ | 2 |
| 23 | $CH(CH_3)CH(OCH_3)_2$ | H | $n-C_4H_9$ | 2 |
| 25 | $CH(CH_3)CH$ 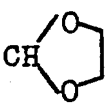 | H | $n-C_4H_9$ | 1 |
| 27 | $CH(CH_3)CH$ 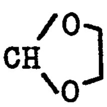 | H | $n-C_4H_9$ | 2 |
| 28 | $CH(CH_3)CH$ 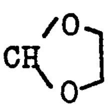 | H | $CH_3$ | 1 |
| 29 | $CH(CH_3)CH$ 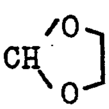 | H | $CH_3$ | 2 |
| 30 | $CH(CH_3)CH$ 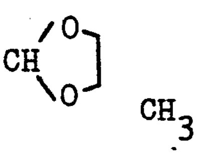 | H | $CH_3$ | 1 |
| 31 | $CH(CH_3)CH$ 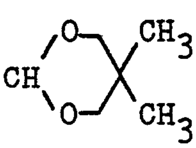 | H | $CH_3$ | 1 |

In entsprechender Weise können folgende Verbindungen hergestellt werden:

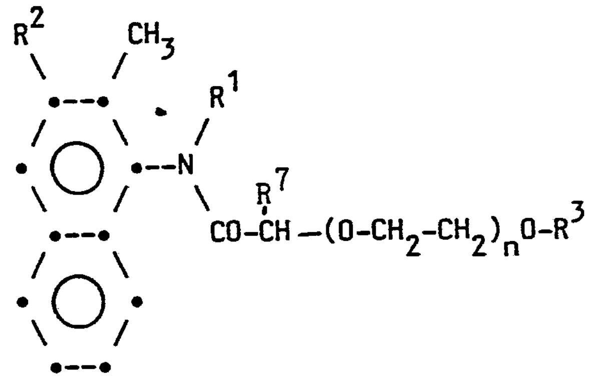

I

| Nr. | $R^1$ | $R^2$ | $R^3$ | n | $n_D^{25}$ | $R^7$ = H |
|---|---|---|---|---|---|---|
| 3 | $CH(CH_3)CO_2iC_3H_7$ | H | $n-C_3H_7$ | 1 | | |
| 4 | $CH(CH_3)CO_2iC_3H_7$ | H | $n-C_4H_9$ | 1 | | |
| 5 | $CH(CH_3)CO_2CH_3$ | $CH_3$ | $n-C_4H_9$ | 1 | | |
| 7 | $CH(CH_3)CO_2CH_3$ | $CH_3$ | $CH_3$ | 2 | | |
| 9 | $CH(CH_3)CO_2iC_3H_7$ | H | $CH_3$ | 2 | | |
| 10 | $CH_3(CH_3CO_2CH_3$ | H | $n-C_3H_7$ | 2 | | |
| 12 | 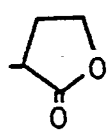 | H | $n-C_3H_7$ | 1 | | |
| 13 | 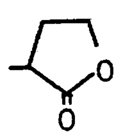 | H | $n-C_4H_9$ | 1 | | |
| 15 | 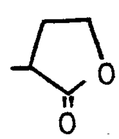 | H | $C_2H_5$ | 2 | | |

| | | | | |
|---|---|---|---|---|
| 16 | 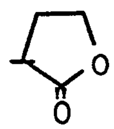 | H | $n\text{-}C_3H_7$ | 2 |
| 17 | 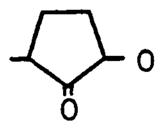 | H | $n\text{-}C_4H_9$ | 2 |
| 19 | $CH(CH_3)CH(OCH_3)_2$ | H | $C_2H_5$ | 1 |
| 20 | $CH(CH_3)CH(OCH_3)_2$ | H | $nC_3H_7$ | 1 |
| 21 | $CH(CH_3)CH(OCH_3)_2$ | H | $n\text{-}C_4H_9$ | 1 |
| 22 | $CH(CH_3)CH(OCH_3)_2$ | H | $CH_3$ | 2 |
| 23 | $CH(CH_3)CH(OCH_3)_2$ | H | $n\text{-}C_4H_9$ | 2 |
| 25 | $CH(CH_3)CH$ 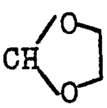 | H | $n\text{-}C_4H_9$ | 1 |
| 27 | $CH(CH_3)CH$ 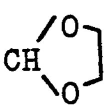 | H | $n\text{-}C_4H_9$ | 2 |
| 28 | $CH(CH_3)CH$ 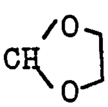 | H | $CH_3$ | 1 |
| 29 | $CH(CH_3)CH$ 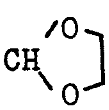 | H | $CH_3$ | 2 |
| 30 | $CH(CH_3)CH$ 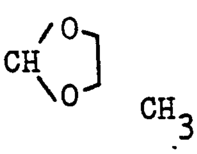 | H | $CH_3$ | 1 |
| 31 | $CH(CH_3)CH$ 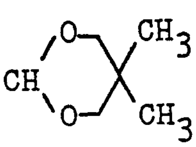 | H | $CH_3$ | 1 |

Die neuen Wirkstoffe zeigen eine starke fungitoxische Wirksamkeit gegen phytopathogene Pilze, insbesondere aus der Klasse der Phycomyceten. Die neuen Verbindungen sind daher beispielsweise geeignet zur Bekämpfung von Phytophthora infestans an Tomaten und Kartoffeln, Phytophthora parasitica an Erdbeeren, Phytophthora cactorum an Äpfeln, Pseudoperonospora cubensis an Gurken, Pseudoperonospora humuli an Hopfen, Peronospora destructor an Zwiebeln, Peronospora sparsa an Rosen, Peronospora tabacina an Tabak, Plasmopara viticola an Reben, Plasmopara halstedii an Sonnenblumen, Sclerospora macrospora an Mais, Bremia lactucae an Salat, Mucor mucedo an Früchten, Phizopus nigricans an Rüben, Erysiphe graminis an Getreide, Uncinula necator an Reben, Podophaera leucotricha an Äpfeln, Sphaerotheca fuliginea an Rosen, Erysiphe cichoriacearum an Gurken. Die fungiziden Mittel enthalten 0,1 bis 95 % (Gewichtsprozent) Wirkstoff, vorzugsweise 0,5 bis 90 %. Die Aufwandmengen liegen je nach Art des gewünschten Effektes zwischen 0,1 und 5 kg Wirkstoff je ha. Ein Teil der Wirkstoffe zeigt kurative Eigenschaften, d.h. die Anwendung der Mittel kann noch nach erfolgter Infektion der Pflanzen durch die Krankheitserreger vorgenommen werden, um einen sicheren Bekämpfungserfolg zu erzielen.

Darüber hinaus sind viele der neuen Verbindungn systemisch wirksam, so daß über die Wurzelbehandlung auch ein Schutz oberirdischer Pflanzenteile möglich ist.

Die neuen Wirkstoffe können auch zusammen mit anderen Wirkstoffen, z.B. Herbiziden, Insektiziden, Wachstumsregulatoren und Fungiziden oder auch mit Düngemitteln vermischt und ausgebracht werden. In vielen Fällen erhält man bei der Mischung mit Fungiziden auch eine Vergrößerung des fungiziden Wirkungsspektrums; bei einer Anzahl dieser Fungizidmischungen treten auch synergistische Effekte auf,

d.h. die fungizide Wirksamkeit des Kombinationsproduktes ist größer als die der addierten Wirksamkeiten der Einzelkomponenten. Eine besonders günstige Vergrößerung des Wirkungsspektrums wird mit folgenden Fungiziden erzielt:

Manganethylenbisdithiocarbamat
Mangan-Zinkethylenbisdithiocarbamat
Ammoniak-Komplex von Zink-(N,N-ethylen-bis-dithiocarbamat)
N-Trichlormethylthio-tetrahydrophthalimid
N-Trichlormethyl-phthalimid
5-Ethoxy-3-trichlormethyl-1,2,3-thiadiazol
2-Methoxycarbonylamino-benzimidazol
2-Thodanmethylthiobenzthiazol
1,4-Dichlor-2,5-dimethoxybenzol
2,3-Dichlor-6-methyl-1,4-oxathiin-5-carbonsäureanilid
2-Methyl-5,6-dihydro-4-H-pyran-3-carbonsäure-anilid
2,4,5-Trimethyl-furan-3-carbonsäureanilid
2-Methyl-furan-3-carbonsäureanilid
2,5-Dimethyl-furan-3-carbonsäurecyclohexylamid
N-Cyclohexyl-N-methoxy-2,5-dimethyl-furan-3-carbonsäure-amid
5-Methyl-5-vinyl-3-(3,5-dichlorphenyl)-2,4-dioxo-1,3-oxazolidin
3-(3,5-Dichlorphenyl)-5-methyl-5-methoxymethyl-1,3-oxazolidin-2,4-dion

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Wirkstoffen kombiniert werden können sind beispielsweise:

Dithiocarbamate und deren Derivate, wie
Ferridimethyldithiocarbamat
Zinkdimethyldithiocarbamat
Zinkethylenbisdithiocarbamat
Tetramethylthiuramdisulfide
Zink-(N,N-propylen-bis-dithiocarbamat
Ammoniak-Komplex von Zink-(N,N'-propylen-bis-dithiocarbamat) und
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat

heterocyclische Strukturen, wie
2-Heptadecyl-2-imidazolin-acetat
2,4-Dichlor-6-(o-chloranilino)-s-triazin
O,O-Diethyl-phthalimidophonothioat
5-Amino-1-(bis-(dimethylamino)-phosphinyl)-3-phenyl-1,2,4-triazol)
2,3-Dicyano-1,4-dithiaanthrachinon
2-Thio-1,3-dithio-(4,5,-b)-chinoxalin
1-(Butylcarbamoyl)-2-benzimidazol-carbaminsäuremethylester
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin-4,4-dioxid
2,3-Dihydro-5-carboxanilido-6-methyl-1,4-oxathiin
2-(Furyl-(2))-benzimidazol
Piperazin-1,4-di-yl-bis-(1-(2,2,2-trichlor-ethyl)-formamid
2-(Thiazolyl-(4)-benzimidazol
5-Butyl-2-dimethylamino-4-hydroxy-6-methyl-pyrimidin

Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol
sowie verschiedene Fungizide, wie
Dodecylguanidinacetat
3-(3-(3,5-Dimethyl-2-oxycyclohexyl)-2-hydroxyethyl)-glutarimid
Hexachlorbenzol
N-Dichlorfluormethylthio-N',N'-dimethyl-N-phenyl-schwefelsäurediamid
2,5-Dimethyl-furan-3-carbonsäureanilid
2-Methyl-genzoesäure-anilid
2-Jod-benzoesäure-anilid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
1-(4-Chlorphenoxy)-3,3-dimethyl-1-(1H-1,2,4-triazol-1-yl)--2-butanon
1-(4-Chlorphenoxy)-3,3-dimethyl-1-(1H-1,2,4-triazol-1-yl)--2-butanol
α-(2-Chlorphenyl)-α-(4-chlorphenyl)-5-pyrimidin-methanol.

Die neuen Wirkstoffe werden beispielsweise in Form von direkt versprühbaren Lösungen, Pulvern, Suspensionen, auch hochprozentige wäßrige, ölige oder sonstige Suspensionen oder Dispersionen, Emulsionen, Öldispersionen, Pasten, Stäubemitteln, Streumitteln, Granulaten, durch Versprühen, Vernebeln, Verstäuben, Verstreuen, Beizen oder Gießen angewendet. Die Aufwendungsformen richten sich ganz nach den Verwendungszwecken; sie sollten in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate z.B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind. Als oberflächenaktive Stoffe kommen in Betracht:
Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole Octadecanole, Salze von sulfatiertem Fettalkoholglykolether, Kondensationsprodukte von sulfoniertem Naphthalin und Naphthalinderivaten mit Formaldehyd, Kon-

densationsprodukte des Naphthalins bzw. der Naphthalinsulfonsäuren mit Phenol und Formaldehyd, Polyoxyethylenoctylphenolether, ethoxyliertes Isooctylphenol-, Octylphenol-, Nonylphenol, Alkylphenolpolyglykolether, Tributylphenylpolyglykolether, Alkalarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehle, Cellulosepulver und andere feste Trägerstoffe.

Für die folgenden Versuche wurden als bekannte Vergleichswirkstoffe die folgenden Verbindungen verwendet.

N-(2,6-Dimethylphenyl)-N-furyl-(2)-alaninsäuremethylester (A)

N-Trichlormethylthio-phthalimid (Verbindung B)

Versuch 1

Wirksamkeit gegen Phytophthora infestans an Tomaten

Blätter von Topfpflanzen der Sorte "Große Fleischtomate" wurden mit wäßriger Spritzbrühe, die 80 % (gew.-%) Wirkstoff und 20 % Emulgiermittel in der Trockensubstanz enthielt, besprüht. Nach dem Antrocknen des Spritzbelages wurden die Blätter mit einer Zoosporenaufschwemmung des Pilzes Phytophthora infestans infiziert. Die Pflanzen wurden dann in einer wasserdampfgesättigten Kammer bei Temperaturen zwischen 16 und 18°C aufgestellt. Nach 5 Tagen hatte sich die Krankheit auf den unbehandelten, jedoch infizierten Kontrollpflanzen so stark entwickelt, daß die fungizide Wirksamkeit der Substanzen beurteilt werden konnte.

Das Ergebnis zeigt, daß beispielsweise die Verbindungen 1, 2, 6, 8, 11 und 18 bei Anwendung als 0,025%ige Wirkstoffbrühe eine bessere fungizide Wirkung (beispielsweise 100 %) haben als der bekannte Vergleichswirkstoff A (beispielsweise 70 %).

Versuch 2

Wirksamkeit gegen Plasmopara viticola

Blätter von Topfreben der Sorte "Müller-Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 10 Tage im Gewächshaus aufgestellt. Erst dann wurden die Blätter mit einer Zoosporenaufschwemmung von Plasmopara

viticola (Rebenperonospora) infiziert. Danach wurden die Reben zunächst für 16 Stunden in einer wasserdampfgesättigten Kammer bei 24°C und anschließend für 8 Tage in einem Gewächshaus mit Temperaturen zwischen 20 und 30°C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis zeigt, daß beispielsweise die Wirkstoffe 1, 2, 6, 8 und 18 bei Anwendung als 0,025%ige Wirkstoffbrühe eine bessere fungizide Wirkung haben (beispielsweise 100 %) als der bekannte Vergleichswirkstoff B (beispielsweise 90 %).

Beispiele für Zubereitungen sind:

I. Man vermischt 90 Gew.-Teile der Verbindung 1 mit 10 Gew.-Teilen N-Methyl-alpha-pyrrolidon und erhält eine Lösung, die zur Anwendung in Form kleinster Tropfen geeignet ist.

II. 20 Gew.-Teile der Verbindung 2 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-mono-ethanolamin, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung 8 werden in einer Mischung gelöst, die aus 40 Gew.-Teilen Cyclohexanon,

30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung 8 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhlt man eine wäßrige Dispersion.

V. 80 Gew.-Teile der Verbindung 11 werden mit 3 Gew.--Teilen des Natriumsalzes der Diisobutylnaphthalin--alpha-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung 18 werden mit 97 Gew.--Teilen feinteiligem Kaolin innig vermischt. Man erhält auf diese Weise ein Stäubemittel, das 3 Gew.-% des Wirkstoffs enthält.

VII. 30 Gew.-Teile der Verbindung 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhlt auf diese Weise eine Aufbereitung des Wirkstoffs mit guter Haftfähigkeit.

VIII. 40 Gew.-Teile der Verbindung 2 werden mit 10 Teilen Natriumsalz eines Phenolsulfonsäure-harnstoff-formaldehyd-Kcndensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung 6 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

## Patentansprüche

1. Glykoletheressigsäurenaphthylamid der Formel

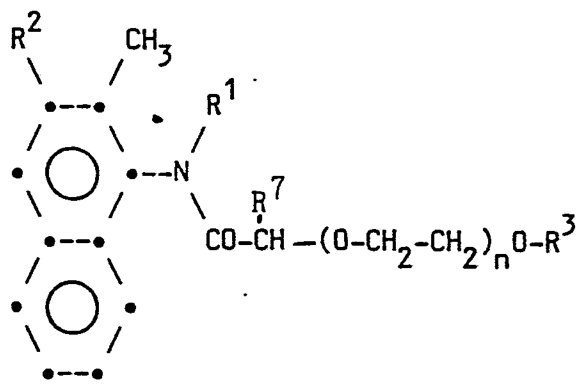

I

worin

$R^1$ $CH(CH_3)CO_2R^4$ oder 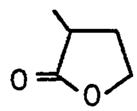

$R^2$ Wasserstoff oder Methyl

$R^4$ $C_1$- bis $C_3$-Alkyl

$R^7$ Wasserstoff oder Methyl

n 1 oder 2

bedeutet, wobei, wenn

n 1 ist, $R^3$ $C_3$- bis $C_6$-Alkyl ist oder wenn n 2 ist, $R^3$ $C_1$- bis $C_6$-Alkyl ist,

$R^1$ bedeutet ferner 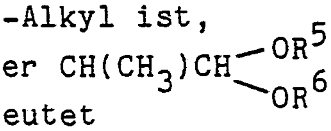

in diesem Fall bedeutet

$R^2$ Wasserstoff oder Methyl

$R^3$ $C_1$- bis $C_6$-Alkyl

$R^5$ und $R^6$ unabhängig voneinander $C_1$- bis $C_6$-Alkyl oder zusammen eine gegebenenfalls mit bis zu 2 Methyl- oder Ethylresten substituierte Alkylenkette mit 2 oder 3 C-Atomen im Alkylenrest

$R^7$ Wasserstoff oder Methyl

n 1 oder 2.

2. Fungizid, enthaltend ein Glykoletheressigsäurenaphthylamid der Formel

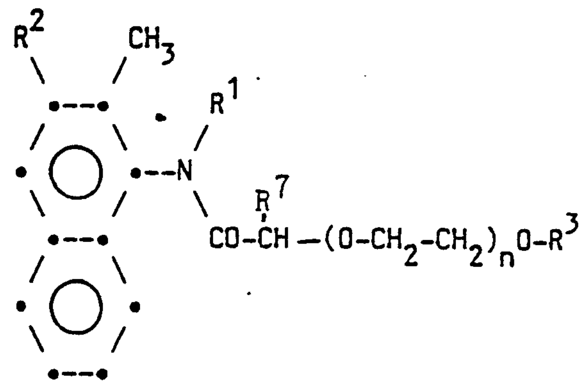

I

worin

$R^1$ $CH(CH_3)CO_2R^4$ oder 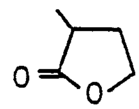

$R^2$ Wasserstoff oder Methyl

$R^4$ $C_1$- bis $C_3$-Alkyl

$R^7$ Wasserstoff oder Methyl

n 1 oder 2

bedeutet, wobei, wenn

n 1 ist, $R^3$ $C_3$- bis $C_6$-Alkyl ist oder wenn n 2 ist, $R^3$ $C_1$- bis $C_6$-Alkyl ist,

$R^1$ bedeutet ferner 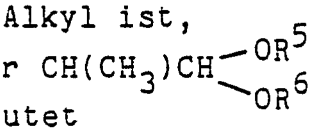

in diesem Fall bedeutet

$R^2$ Wasserstoff oder Methyl

$R^3$ $C_1$- bis $C_6$-Alkyl

$R^5$ und $R^6$ unabhängig voneinander $C_1$- bis $C_6$-Alkyl oder zusammen eine gegebenenfalls mit bis zu 2 Methyl- oder Ethylresten substituierte Alkylenkette mit 2 oder 3 C-Atomen im Alkylenrest

$R^7$ Wasserstoff oder Methyl

n 1 oder 2.

3. Fungizid, enthaltend einen festen oder flüssigen Trägerstoff und ein Glykoletheressigsäurenaphthylamid der Formel

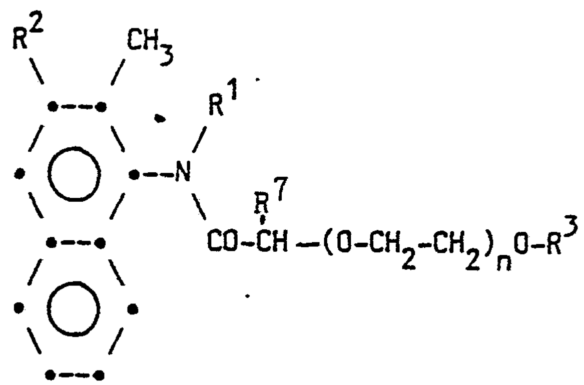

I

worin

$R^1$ $CH(CH_3)CO_2R^4$ oder 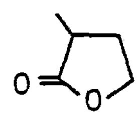

$R^2$ Wasserstoff oder Methyl

$R^4$ $C_1$- bis $C_3$-Alkyl

$R^7$ Wasserstoff oder Methyl

n 1 oder 2

bedeutet, wobei, wenn

n 1 ist, $R^3$ $C_3$- bis $C_6$-Alkyl ist oder wenn n 2 ist, $R^3$ $C_1$- bis $C_6$-Alkyl ist,

$R^1$ bedeutet ferner $CH(CH_3)CH\begin{smallmatrix}OR^5\\OR^6\end{smallmatrix}$

in diesem Fall bedeutet

$R^2$ Wasserstoff oder Methyl

$R^3$ $C_1$- bis $C_6$-Alkyl

$R^5$ und $R^6$ unabhängig voneinander $C_1$- bis $C_6$-Alkyl oder zusammen eine gegebenenfalls mit bis zu 2 Methyl- oder Ethylresten substituierte Alkylenkette mit 2 oder 3 C-Atomen im Alkylenrest

$R^7$ Wasserstoff oder Methyl

n 1 oder 2.

4. Verfahren zur Herstellung eines Fungizids, <u>dadurch gekennzeichnet</u>, daß man einen festen oder flüssigen Trägerstoff vermischt mit einem Glykoletheressigsäurenaphthylamid der Formel

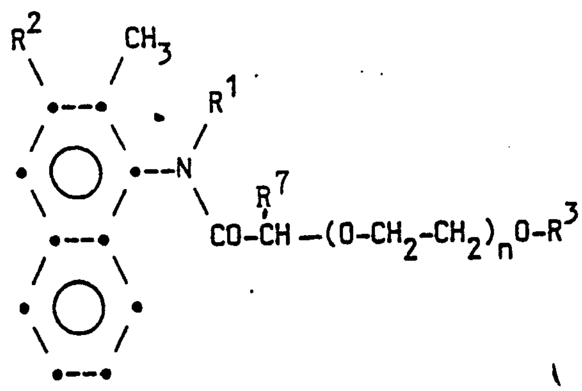 I

worin

$R^1$ $CH(CH_3)CO_2R^4$ oder 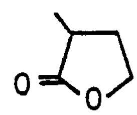

$R^2$ Wasserstoff oder Methyl

$R^4$ $C_1$- bis $C_3$-Alkyl

$R^7$ Wasserstoff oder Methyl

n 1 oder 2

bedeutet, wobei, wenn

n 1 ist, $R^3$ $C_3$- bis $C_6$-Alkyl ist oder wenn n 2 ist, $R^3$ $C_1$- bis $C_6$-Alkyl ist,

$R^1$ bedeutet ferner $CH(CH_3)CH\begin{matrix}OR^5\\OR^6\end{matrix}$

in diesem Fall bedeutet

$R^2$ Wasserstoff oder Methyl

$R^3$ $C_1$- bis $C_6$-Alkyl

$R^5$ und $R^6$ unabhängig voneinander $C_1$- bis $C_6$-Alkyl oder zusammen eine gegebenenfalls mit bis zu 2 Methyl- oder Ethylresten substituierte Alkylenkette mit 2 oder 3 C-Atomen im Alkylenrest

$R^7$ Wasserstoff oder Methyl

n 1 oder 2.

5. Verfahren zur Bekämpfung von Pilzen, <u>dadurch gekennzeichnet</u>, daß man die Pilze oder die vor Pilzbefall zu schützenden Gegenstände behandelt mit einem Glykoletheressigsäurenaphthylamid der Formel

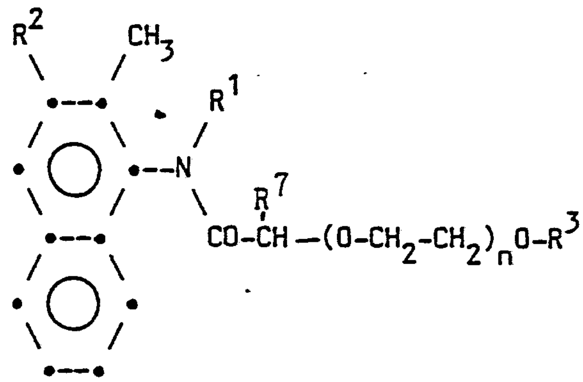 I

worin

$R^1$ $CH(CH_3)CO_2R^4$ oder 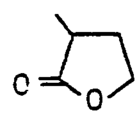

$R^2$ Wasserstoff oder Methyl

$R^4$ $C_1$- bis $C_3$-Alkyl

$R^7$ Wasserstoff oder Methyl

n 1 oder 2

bedeutet, wobei, wenn

n 1 ist, $R^3$ $C_3$- bis $C_6$-Alkyl ist oder wenn n 2 ist, $R^3$ $C_1$- bis $C_6$-Alkyl ist,

$R^1$ bedeutet ferner 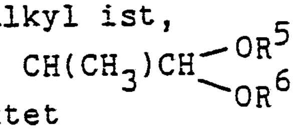

in diesem Fall bedeutet

$R^2$ Wasserstoff oder Methyl

$R^3$ $C_1$- bis $C_6$-Alkyl

$R^5$ und $R^6$ unabhängig voneinander $C_1$- bis $C_6$-Alkyl oder zusammen eine gegebenenfalls mit bis zu 2 Methyl- oder Ethylresten substituierte Alkylenkette mit 2 oder 3 C-Atomen im Alkylenrest

$R^7$ Wasserstoff oder Methyl

n 1 oder 2.

6. Verfahren zur Herstellung eines Glykoletheressigsäurenaphthylamids der Formel

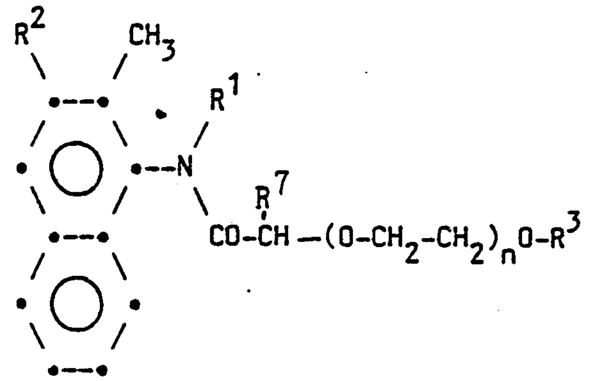

I

worin

$R^1$ $CH(CH_3)CO_2R^4$ oder 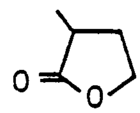

$R^2$ Wasserstoff oder Methyl

$R^4$ $C_1$- bis $C_3$-Alkyl

$R^7$ Wasserstoff oder Methyl

n 1 oder 2

bedeutet, wobei, wenn

n 1 ist, $R^3$ $C_3$- bis $C_6$-Alkyl ist oder wenn n 2 ist, $R^3$ $C_1$- bis $C_6$-Alkyl ist,

$R^1$ bedeutet ferner $CH(CH_3)CH\genfrac{}{}{0pt}{}{OR^5}{OR^6}$

in diesem Fall bedeutet

$R^2$ Wasserstoff oder Methyl

$R^3$ $C_1$- bis $C_6$-Alkyl

$R^5$ und $R^6$ unabhängig voneinander $C_1$- bis $C_6$-Alkyl oder zusammen eine gegebenenfalls mit bis zu 2 Methyl- oder Ethylresten substituierte Alkylenkette mit 2 oder 3 C-Atomen im Alkylenrest

$R^7$ Wasserstoff oder Methyl

n 1 oder 2,

dadurch gekennzeichnet, daß man ein Naphthylamin der Formel

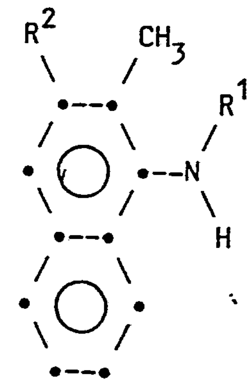

II

in welcher $R^1$ und $R^2$ die im Anspruch 1 angegebenen Bedeutungen haben mit einer Glykolethersäure der Formel

$$HO-CO-\overset{R^{7}}{\overset{|}{C}}H-(O-CH_2-CH_2)_n O-R^3 \qquad III$$

oder ihrem Säurehalogenid umsetzt, wobei $R^3$, $R^7$ und n die im Anspruch 1 angegebenen Bedeutungen haben.

7. Verbindung gemäß Anspruch 1, dadurch gekennzeichnet, daß $R^1$ 1-Methoxycarbonylethyl, $R^2$ Wasserstoff, $R^7$ Wasserstoff, $R^3$ $C_3$-$C_4$-Alkyl und n 1 oder 2 bedeutet.

8. Fungizid enthaltend eine Verbindung definiert wie in Anspruch 1, dadurch gekennzeichnet, daß $R^1$ 1-Methoxycarbonylethyl, $R^2$ und $R^7$ Wasserstoff, $R^3$ $C_3$-$C_4$-Alkyl und n 1 oder 2 bedeutet.

## Patentansprüche (für Österreich)

1. Fungizid enthaltend ein Glykoletheressigsäurenaphthylamid der Formel

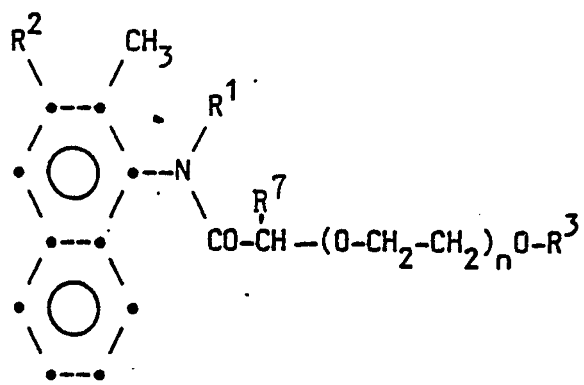

I

worin

$R^1$ $CH(CH_3)CO_2R^4$ oder 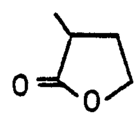

$R^2$ Wasserstoff oder Methyl

$R^4$ $C_1$- bis $C_3$-Alkyl

$R^7$ Wasserstoff oder Methyl

n 1 oder 2

bedeutet, wobei, wenn

n 1 ist, $R^3$ $C_3$- bis $C_6$-Alkyl ist oder wenn n 2 ist, $R^3$ $C_1$- bis $C_6$-Alkyl ist,

$R^1$ bedeutet ferner $CH(CH_3)CH$ 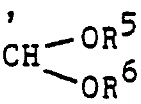

in diesem Fall bedeutet

$R^2$ Wasserstoff oder Methyl

$R^3$ $C_1$- bis $C_6$-Alkyl

$R^5$ und $R^6$ unabhängig voneinander $C_1$- bis $C_6$-Alkyl oder zusammen eine gegebenenfalls mit bis zu 2 Methyl- oder Ethylresten substituierte Alkylenkette mit 2 oder 3 C-Atomen im Alkylenrest

$R^7$ Wasserstoff oder Methyl

n 1 oder 2.

2. Fungizid, enthaltend einen festen oder flüssigen Trägerstoff und ein Glykoletheressigsäurenaphthylamid der Formel

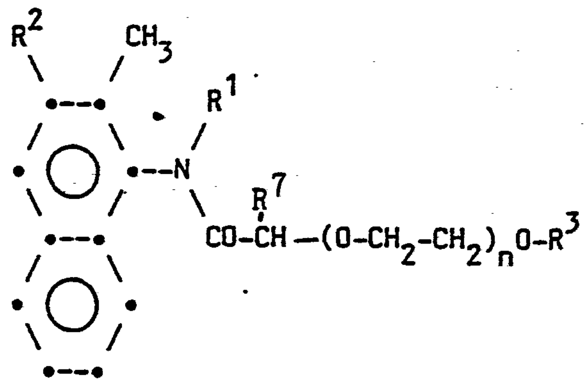

I

worin

$R^1$ $CH(CH_3)CO_2R^4$ oder 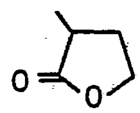

$R^2$ Wasserstoff oder Methyl

$R^4$ $C_1$- bis $C_3$-Alkyl

$R^7$ Wasserstoff oder Methyl

n 1 oder 2

bedeutet, wobei, wenn

n 1 ist, $R^3$ $C_3$- bis $C_6$-Alkyl ist oder wenn n 2 ist, $R^3$ $C_1$- bis $C_6$-Alkyl ist,

$R^1$ bedeutet ferner $CH(CH_3)CH$ 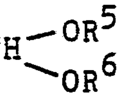

in diesem Fall bedeutet

$R^2$ Wasserstoff oder Methyl

$R^3$ $C_1$- bis $C_6$-Alkyl

$R^5$ und $R^6$ unabhängig voneinander $C_1$- bis $C_6$-Alkyl oder zusammen eine gegebenenfalls mit bis zu 2 Methyl- oder Ethylresten substituierte Alkylenkette mit 2 oder 3 C-Atomen im Alkylenrest

$R^7$ Wasserstoff oder Methyl

n 1 oder 2.

3. Verfahren zur Herstellung eines Fungizids, <u>dadurch gekennzeichnet</u>, daß man einen festen oder flüssigen Trägerstoff vermischt mit einem Glykoletheressigsäurenaphthylamid der Formel

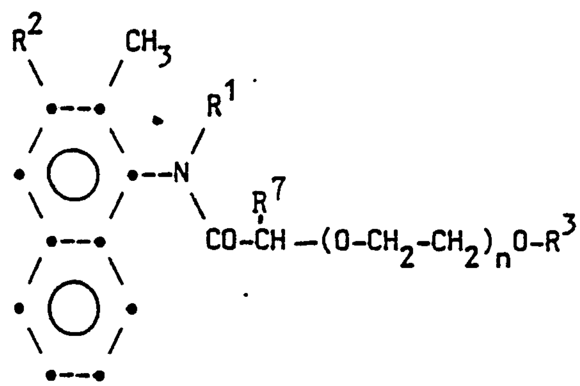

I

worin

$R^1$ $CH(CH_3)CO_2R^4$ oder 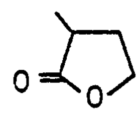

$R^2$ Wasserstoff oder Methyl

$R^4$ $C_1$- bis $C_3$-Alkyl

$R^7$ Wasserstoff oder Methyl

n 1 oder 2

bedeutet, wobei, wenn

n 1 ist, $R^3$ $C_3$- bis $C_6$-Alkyl ist oder wenn n 2 ist, $R^3$ $C_1$- bis $C_6$-Alkyl ist,

$R^1$ bedeutet ferner 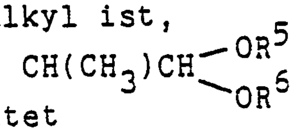

in diesem Fall bedeutet

$R^2$ Wasserstoff oder Methyl

$R^3$ $C_1$- bis $C_6$-Alkyl

$R^5$ und $R^6$ unabhängig voneinander $C_1$- bis $C_6$-Alkyl oder zusammen eine gegebenenfalls mit bis zu 2 Methyl- oder Ethylresten substituierte Alkylenkette mit 2 oder 3 C-Atomen im Alkylenrest

$R^7$ Wasserstoff oder Methyl

n 1 oder 2.

4. Verfahren zur Bekämpfung von Pilzen, <u>dadurch gekennzeichnet</u>, daß man die Pilze oder die vor Pilzbefall zu schützenden Gegenstände behandelt mit einem Glykoletheressigsäurenaphthylamid der Formel

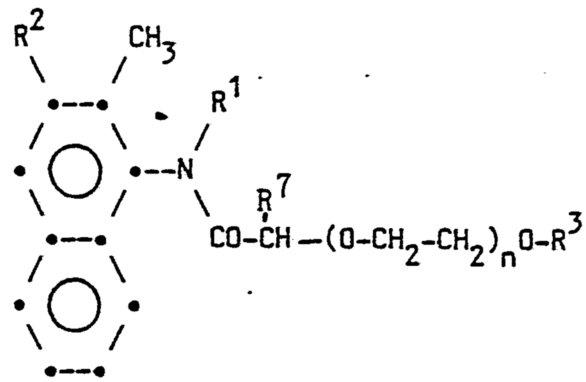 I

worin

$R^1$ $CH(CH_3)CO_2R^4$ oder 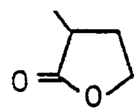

$R^2$ Wasserstoff oder Methyl

$R^4$ $C_1$- bis $C_3$-Alkyl

$R^7$ Wasserstoff oder Methyl

n 1 oder 2

bedeutet, wobei, wenn

n 1 ist, $R^3$ $C_3$- bis $C_6$-Alkyl ist oder wenn n 2 ist, $R^3$ $C_1$- bis $C_6$-Alkyl ist,

$R^1$ bedeutet ferner $CH(CH_3)CH$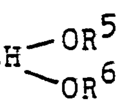

in diesem Fall bedeutet

$R^2$ Wasserstoff oder Methyl

$R^3$ $C_1$- bis $C_6$-Alkyl

$R^5$ und $R^6$ unabhängig voneinander $C_1$- bis $C_6$-Alkyl oder zusammen eine gegebenenfalls mit bis zu 2 Methyl- oder Ethylresten substituierte Alkylenkette mit 2 oder 3 C-Atomen im Alkylenrest

$R^7$ Wasserstoff oder Methyl

n 1 oder 2.

5\. Verfahren zur Herstellung eines Glykoletheressigsäure-naphthylamids der Formel

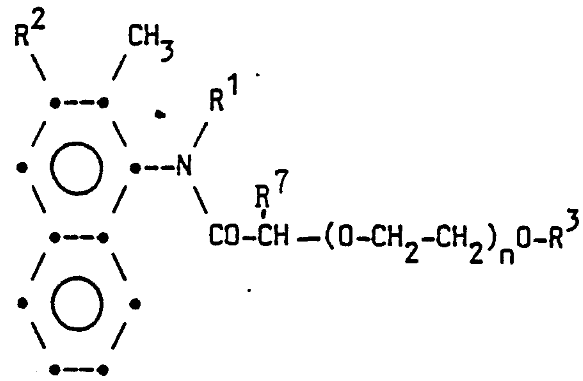

I

worin

$R^1$ $CH(CH_3)CO_2R^4$ oder 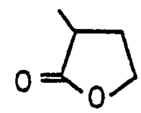

$R^2$ Wasserstoff oder Methyl

$R^4$ $C_1$- bis $C_3$-Alkyl

$R^7$ Wasserstoff oder Methyl

n 1 oder 2

bedeutet, wobei, wenn

n 1 ist, $R^3$ $C_3$- bis $C_6$-Alkyl ist oder wenn n 2 ist, $R^3$ $C_1$- bis $C_6$-Alkyl ist,

$R^1$ bedeutet ferner $CH(CH_3)CH$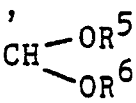

in diesem Fall bedeutet

$R^2$ Wasserstoff oder Methyl

$R^3$ $C_1$- bis $C_6$-Alkyl

$R^5$ und $R^6$ unabhängig voneinander $C_1$- bis $C_6$-Alkyl oder zusammen eine gegebenenfalls mit bis zu 2 Methyl- oder Ethylresten substituierte Alkylenkette mit 2 oder 3 C-Atomen im Alkylenrest

$R^7$ Wasserstoff oder Methyl

n 1 oder 2,

dadurch gekennzeichnet, daß man ein Naphthylamin der Formel

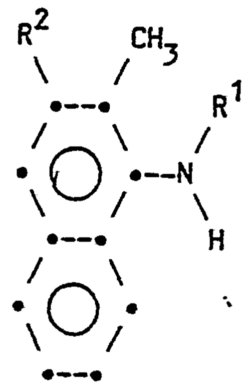

II

in welcher $R^1$ und $R^2$ die im Anspruch 1 angegebenen Bedeutungen haben mit einer Glykolethersäure der Formel

$$HO-CO-\overset{R^7}{CH}-(O-CH_2-CH_2)_nO-R^3 \qquad III$$

oder ihrem Säurehalogenid umsetzt, wobei $R^3$, $R^7$ und n die im Anspruch 1 angegebenen Bedeutungen haben.

6. Fungizid enthaltend eine Verbindung definiert wie in Anspruch 1, dadurch gekennzeichnet, daß $R^1$ 1-Methoxycarbonylethyl, $R^2$ und $R^7$ Wasserstoff, $R^3$ $C_3$-$C_4$-Alkyl und n 1 oder 2 bedeutet.

Europäisches Patentamt

## EUROPÄISCHER RECHERCHENBERICHT

0069293

Nummer der Anmeldung

EP 82 10 5600

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl. 3) |
| Y | EP-A-0 018 510 (CIBA-GEIGY) *Patentansprüche* | 1-5 | C 07 C 103/38<br>C 07 C 103/48<br>C 07 D 307/32<br>C 07 D 317/28<br>C 07 D 319/06<br>A 01 N 37/22<br>A 01 N 37/46<br>A 01 N 43/08<br>A 01 N 43/24 |
| Y | EP-A-0 029 996 (BASF) *Patentansprüche* | 1-5 | |
| Y | FR-A-2 449 684 (CHEVRON) *Patentansprüche; Beispiel 6* | 1-5 | |
| Y | DE-A-2 939 350 (BASF) *Patentansprüche* | 1-6 | |
| | | | RECHERCHIERTE SACHGEBIETE (Int. Cl. 3) |
| | | | C 07 C 103/00<br>C 07 D 307/00<br>C 07 D 317/00<br>A 01 N 37/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 28-09-1982 | MOREAU J.M. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument